# EUROPEAN PATENT SPECIFICATION

(11) **EP 1 690 566 B1**
(45) Date of publication and mention of the grant of the patent: **09.04.2008**
(21) Application number: 05075347.4
(22) Date of filing: 09.02.2005
(51) Int. Cl.: A61N 1/365, A61B 5/053

(54) **Ischemia detector**
Ischämiedetektor
Détecteur d' ischémie

(43) Date of publication of application: 16.08.2006
(73) Proprietor: Chirife, Raul, 1641 Buenos Aires (AR)
(72) Inventor: Chirife, Raul, 1641 Buenos Aires (AR)
(74) Representative: Vinci, Marcello

(56) References cited:
- EP-A- 0 788 812
- EP-A- 0 879 618
- EP-A- 1 384 433
- US-A- 5 282 840

## Description

The invention relates to an ischemia detection apparatus comprising an ischemia detector. The invention also relates to an implantable medical device comprising such ischemia detector. The implantable medical device preferably is a heart stimulator such as a cardiac pacemaker, defibrillator, cardioverter or the like. Such medical implantable device becomes an ischemia detection apparatus by virtue of such ischemia detector.

The ischemia detection apparatus comprises an impedance measuring stage being connected to an electrode lead connector. Said electrode lead connector is adapted to be connected to at least two intracardiac and/or epicardial electrodes and to produce an impedance signal indicative of a measured impedance between said at least two electrodes. When operating the Ischemia detection apparatus the measured impedance is an intracardiac impedance which depends to a major extend on the blood filling of a heart's chamber like a right or a left ventricle of the heart.

An ischemia detector is connected to the impedance measuring stage and a memory for impedance signal values. The ischemia detector is adapted to determine the presence of an ischemia by evaluating measured intracardiac impedance values.

Ischemia detection apparatuses or implantable medical devices (IMDs) incorporating an ischemia detector responsive to a measured intracardiac impedance are know from the prior art, see patents US 6,604,000 or US 6,256,538. Known methods to determine the presence of an acute ischemia based on measured intracardiac impedance values rely the evaluation of magnitude of the impedance waveform during a cardiac cycle as this magnitude reflects the difference the maximum and the minimum of blood filling during one cardiac cycle. Document EP-A-1 384 433 discloses an apparatus according to the preamble of claim 1. The invention seeks to provide for an alternative ischemia detection apparatus and an alternative ischemia detector responsive to intracardiac impedance values.

According to the invention, this object is achieved by an ischemia detector in an ischemia detection apparatus or an implantable medical device (IMD) as set out in the introduction, wherein the ischemia detector is adapted to respond to a change in an impedance signal reflected the endsystolic impedance of at least two different cardiac cycles.

The ischemia detector according to the invention responds to the ratio of or the difference between an actual endsystolic impedance value and a stored reference endsystolic impedance value, respectively. Both the ratio or the difference between an actual and a stored impedance value are characteristic for a change of endsystolic impedance.

As further discussed later in this document, the invention is based on the the fact, that a change in endsystolic impedance alone can be used to detect the onset of ischemia.

The ischemia detector compares an actual endsystolic impedance value to an reference impedance value depending on at least one past endsystolic impedance value.

For comparing the actual impedance value with the reference impedance value the ischemia detector comprises a comparator, said comparator being adapted to compare an actual endsystolic impedance value to a stored impedance value.

The comparator is connected to a timer, said timer being adapted to determine the duration of a time period during which the ratio or difference between the actual endsystolic impedance value and the stored impedance value exceeds the preset threshold value. Only if the ratio or the difference between the actual and the stored endsystolic impedance value exceeds the preset threshold value for more than a preset time period, an ischemia signal reflecting the detection of cardiac ischemia is generated by the ischemia detector or the ischemia detecting apparatus, respectively. The time may be of the time out type, which generates a time-out signal if net reset before a preset time. In such embodiment, the time out timer would be started by the comparator, if the ratio or the difference between the actual and the stored impedance value exceeds the preset threshold. The timer would be reset, if the comparator detects that the ratio or the difference between the actual and the stored impedance value becomes smaller than the preset threshold value while the timer is running. If the timer runs out prior to its timeout, that is if the timer is not reset or stopped by the comparator prior to time out, a time out signal is generated which corresponds to a ischemia signal, or, which in fact is the ischemia signal. It will be appreciated that such ischemia detector can easily be realized by the man skilled in the art.

As an endsystolic impedance value for a single cardiac cycle the maximum value of the measured intracardic impedance during the cardiac cycle can be taken as will be set out later in this document. Therefore, in a preferred embodiment the ischemia detector is adapted to determine an endsystolic impedance value for cardiac cycle by determining the maximum impedance measured by the impedance measuring stage during a cardiac cycle.

Preferably, the ischemia detector comprises a long term averaging stage being adapted to determine a long term average value from a plurality of endsystolic impedance values, the long term averaging stage being connected to a long term endsystolic impedance average memory. Said long term average impedance value preferably constitutes the reference impedance value, which is to be compared to an actual impedance value in the preferred embodiment.

In the preferred embodiment, the ischemia detector comprises a short term averaging stage being adapted to determine a short term average value from a plurality of endsystolic impedance values, the short term averaging stage being connected to a short term endsystolic impedance average memory. The short term average endsystolic impedance value is preferably used as the actual impedance value to be compared to the reference impedance value in the preferred embodiment.

The stored impedance value forms the reference value and is given by the long term average impedance value. The actual impedance value depends on the short term average impedance value derived from measured endsystolic impedance values determined for a plurality of cardiac cycles.

Both, the short term average endsystolic impedance value und the long term average endsystolic impedance value are preferably calculated as moving averages of measured endsystolic impedance values for a number of cardiac cycles. Of course, the number of cardiac cycles considered to calculate the long term average endsystolic impedance value is larger than the number of cardiac cycles considered to calculate the short term average endsystolic impedance value.

In a preferred embodiment, it is the comparator of the impedance detector, which is adapted to determine whether or not the ratio or the difference between the actual endsystolic impedance value and the stored impedance value exceeds a preset threshold value. Therefore, it can be stated, that the impedance detector according to the invention compares the actual impedance value with stored impedance value, thereby generating a signal reflecting the ratio or he difference between the two values as a result of this kind of comparison. The signal thus generated is than compared to a threshold value for the ratio or the difference in a second kind of comparison. Both the kinds of comparison may be considered as to parts of a more general comparison between the actual (short term) and the stored (long term, reference) endsystolic impedance values.

Further aspects of the invention will become apparent from the following abstract:

The apparatus and method for myocardial ischemia detection according to the invention uses intracardiac impedance. Unlike conventional hemodynamic sensors, the impedance sensor does not require sensor hardware in the leads since it uses conventional pacing-type electrodes. The device could be implantable or external, and uses specific changes in the impedance waveform to detect the onset and end of an ischemic episode. Impedance detection is achieved using a plurality of standard implantable grade electrodes placed within the ventricle, on the surface of the heart (epicardial or coronary transvenous) or any combination thereof. The impedance apparatus is preferably DC-coupled, and the signal is analyzed by an algorithm that takes into account the time course, duration and extent of one or more hemodynamic parameters obtained from cardiac impedance. Ischemia detection by implantable devices could be used to warn the patient to contact his/her doctor, to transmit the event to monitoring devices, to store the event for further analysis and to effect a change in the operating characteristics of the device, such as rate reduction (pacemaker), drug infusion, nerve stimulation and the like.

Now, the background of the invention shall be discussed Ischemia is the imbalance between myocardial oxygen supply and demand. It is generally the result of obstruction of the blood vessels providing nourishment to the heart muscle, the coronary arteries. Obstruction of these blood vessels occurs slowly due to a variety of genetic, dietary, environmental and other causes known as risk factors, and is clinically manifested when the obstruction is severe and when the patient is subject to cardio-circulatory stress, such as emotion, exercise, high blood pressure and/or fast heart rate. All of these cause an increase in the oxygen demands by the myocardium, and since the supply is limited by obstruction of the arteries, the clinical manifestations of ischemia ensue. These are chest pains, arrhythmias, heart failure and eventually a myocardial infarction, which is the permanent damage caused by prolonged, unrelenting ischemia. Although typical chest pain is an easily recognized symptom, this is not always the rule, for some patients may have ischemia without pain. The substrate of ischemia is the partial obstruction of the coronary arteries by an atheromatous plaque, diminishing blood flow to the heart muscle. Coronary arteries may present blockage of about 90 % of the lumen of the vessel without symptoms at rest, due to the coronary flow reserve. Since the coronary arteries may also suffer from spasms that further reduce blood supply, ischemic episodes result from a combination of a fixed obstruction and spasm or spasm alone, which allows ischemia to occur during exercise as well as at rest.

Due to the high prevalence of ischemic heart disease, it is likely that a patient receiving a cardiac pacemaker implant may also suffer from ischemic heart disease, whether it be the typical variety with chest pain, or silent, without symptoms during the ischemic episode. Naturally, as soon as ischemia starts, a reduction of heart rate may prove beneficial to the patient, since heart rate is a major determinant of myocardial oxygen consumption. In fact, most patients with ischemia are treated with beta blockers. These are drugs that by virtue of reducing heart rate and the force of contraction reduce also myocardial oxygen consumption, thus protecting the myocardium.

There are several clinical manifestations of ischemia.

Myocardial ischemia produces numerous clinical, electrocardiographic, hemodynamic, and metabolic manifestations. Typically, the clinical diagnosis of an acute ischemic episode is made by the analysis of the electrocardiogram, myocardial perfusion studies, radionuclide ventriculography, and others. Detection of ischemia from the intracardiac electrogram obtained from pacemakers is not possible, because the right ventricular endocardial electrode will show ischemic changes only when ischemia takes place in the close vicinity of the electrode. With biventricular pacemakers having an additional electrode in the surface of the left ventricle, detection may be slightly improved but still is severely limited, especially because biventricular pacing for the treatment of heart failure is permanent. Since ventricular capture interferes with ischemia detection, pacing may need to be temporarily stopped to visualize the intrinsic, unpaced electrogram for ischemia diagnosis.

There are also several hemodynamic manifestations of ischemia

In the presence of reduced oxygen and nutrient supply, the heart muscle (myocardium) metabolism suffers and important functional changes take place. Both the force of systolic contraction (contractility) and the relaxation properties of the myocardium are adversely affected. Reduction of the force of contraction is manifested by a reduction of cardiac ejection fraction (the ratio between stroke volume and end-diastolic volume) and of cardiac output. Ischemia also affects the relaxation properties of the heart muscle by increasing its stiffness, that is, decreasing compliance. Alterations of the systolic function by ischemia have been documented by numerous publications of studies done in patients with coronary artery disease during exercise, using radionuclide ventriculography. This method allows the non-invasive measurement of global and regional myocardial contractility during exercise. In normal individuals the ejection fraction and cardiac output increase during exercise, while in patients with coronary lesions, at the onset of ischemia during effort there is a prompt and significant decrease in ejection fraction, reversing the normal, non-ischemic behavior. These changes usually appear promptly, and frequently precede the onset of chest pain and of alterations in the electrocardiogram, such as ST segment displacement and/or T wave inversion. These observations abound in the literature, and tests conducted in many patients using radionuclide ventriculography, confirm the value of hemodynamic indices to signal the onset of acute ischemia. Another typical hemodynamic marker of alterations of cardiac diastolic properties is a rise of left ventricular end-diastolic pressure. Also, there is abundant literature demonstrating that ischemia will cause a rise ventricular end-diastolic pressure, whichever the coronary artery or cardiac region is affected. In the clinical setting, left ventricular ischemia is far more common than right ventricular ischemia, but accessing the left ventricular cavity to measure end-diastolic pressure is more difficult and far more risky. Assessment of left ventricular end-diastolic pressure can be made by various non-invasive and invasive procedures. Among the former, the response of the arterial blood pressure during the Valsalva maneuver (forced expiration with closed glotis) marks the presence of an elevated end-diastolic pressure, and using invasive means, the introduction of a pressure catheter in the pulmonary artery through a venous puncture gives an estimate of diastolic left ventricular pressures. This procedure is facilitated by a balloon-tipped catheter (Swan-Ganz) which is guided by blood flow into the desired pulmonary vessel. The pulmonary artery pressure rises during left ventricular ischemia as a consequence of backward transmission of the left ventricular diastolic pressure through the pulmonary veins, capillaries, and branches of the pulmonary artery.

The invention is based on a novel approach for the diagnosis of ischemia using intracardiac impedance sensor

Unpublished studies carried out during percutaneous transcoronary angioplasty (PTCA) or balloon angioplasty of the coronary arteries while recording right ventricular impedance (a marker of ventricular chamber volume) have shown a significant and consistent changes of hemodynamic parameters obtained from the impedance waveform during occlusive balloon inflation in any of the main branches of the coronary arteries. The PTCA procedure is aimed at reopening a critical lesion in one or more coronary vessels by using a small balloon near the tip of a catheter guided by X-rays into the affected coronary artery. Inflation of the balloon causes flattening of the atheromatous plaque and reopening of the lumen, thus re-establishing blood flow. As expected, balloon inflation within a coronary artery the vessel is completely occluded by the balloon, thus causing a temporary ischemic episode similar in its clinical, hemodynamic, and metabolic manifestations to the spontaneous ischemic attack in the ambulatory patient. During balloon inflation patients frequently present typical ECG changes, drop in the arterial blood pressure and/or chest pains.

Whether ischemia is caused by an increase of metabolic demands (due to rate and/or blood pressure rise) or coronary artery spasm (decreased supply), the hemodynamic manifestations are similar. In above mentioned unpublished study an immediate change in impedance-derived right ventricular hemodynamic parameters was observed when any coronary vessel (left anterior descending, circumflex, diagonal branch, right coronary artery and marginal branches) was occluded by balloon inflation. Naturally, balloon inflation in arteries irrigating dead, infarcted myocardium did not cause any hemodynamic change nor any clinical manifestation. Of note is that occlusion of coronary arteries irrigating left ventricular myocardium also caused hemodynamic changes in the right ventricle. Findings are comparable to those observed with radionuclide ventriculography during exercise: reduction in ejection fraction is observed only when viable, although ischemic myocardium is present. Balloon angioplasty for the treatment of blocked coronary arteries thus offered an excellent model to study the hemodynamic manifestations of acute ischemia, and was also used to validate the hypothesis of this invention.

The object of the present invention is a pacemaker, defibrillator or other implantable or external device embodying an impedance sensor sensitive to ischemia that can be used for diagnosis or treatment. In the diagnostic mode the device may warn the patient, transmit a signal via telemetry to monitoring devices and/or store data in the device memory for further analysis by the doctor. In the therapeutic mode ischemia detection may effect a change in pacing parameters, produce nerve stimulation and/or initiate a drug delivery.

The IMD or ischemia detection according to the invention uses cardiac impedance as a hemodynamic marker.

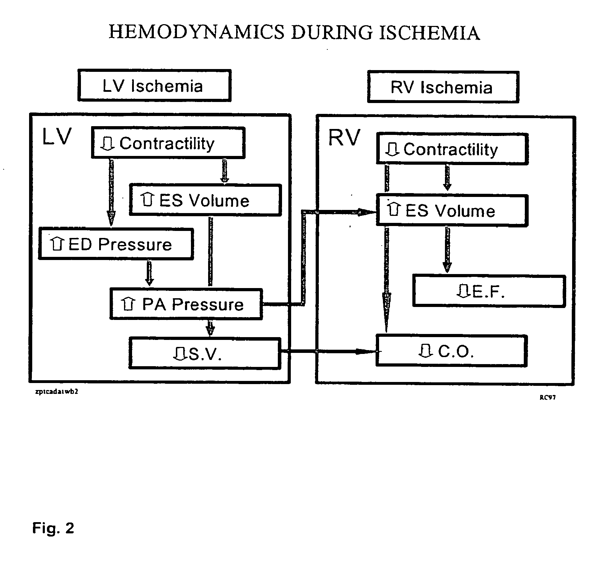

There are numerous publications supporting the value of DC-coupled cardiac impedance as a marker of cardiac preload (right ventricular end-diastolic volume), contractility (ejection fraction, residual volume) and pump function (cardiac output). Above studies of patients during PTCA procedures indicated that DC-coupled intracardiac impedance provides estimates of key hemodynamic variables that are affected, whether the ischemia occurs in the right or left ventricle. These studies revealed that changes of end-systolic systolic impedance, a marker of end-systolic volume (residual volume) are indicators of the onset of ischemia, although the mechanisms by which the changes are apparent may differ depending on the site of ischemia. For example, left ventricular ischemia produced by temporary occlusion of the left anterior descending or diagonal coronary arteries (both irrigating left ventricular myocardium) would cause an immediate change in left ventricular diastolic compliance which in turn would cause a backward rise in the pulmonary veins, pulmonary capillaries and ultimately in the pulmonary artery. A pressure rise in the pulmonary artery causes an immediate change in right ventricular afterload (that is, the load the ventricle has to overcome to eject blood), and as a consequence of this, a rise in right ventricular end-systolic volume. In addition, it is well known that whatever hemodynamic changes take place in the left ventricle will be manifested in the right and *vice-versa.* Therefore, cardiac output changes occurring during left ventricular ischemia will be detected after a few seconds in the right side of the heart. Fig. 2 depicts above mechanisms.

A preferred embodiment of the invention shall now be disclosed with respect to the drawings. The drawings show in
- Fig. 1:: a cardiac pacemaker with an ischemia dtector according to the invention; and
- Fig. 2:: a graphical representation of the hemodynamics during ischemia.

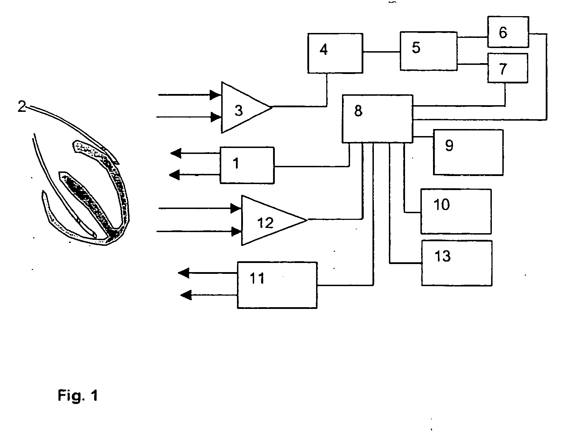

Although there are numerous applications of the ischemia detection sensor, a cardiac pacemaker is described as an example for an ischemia detection apparatus in Fig. 1.

An apparatus comprises:
a) an impedance sensor, formed by an impedance detection circuit
b) a special ischemia detector
c) electrical and/or mechanical means to take an action after ischemia is detected.

In this example a DC-coupled intracardiac impedance sensor is used allowing absolute values of end-diastolic (EDZ) and end-systolic impedance (ESZ) to be measured. The difference between EDZ and ESZ is the stroke impedance, a marker of stroke volume.

The impedance sensor circuit has a carrier oscillator 1 coupled to two or more cardiac electrodes 2, which could be either intraventricular, epicardial or any combination thereof. The epicardial approach could be direct (epicardial electrode) or using a lead passed through a coronary vein. The resulting voltage of two or more of the sensing electrodes is directed to amplifier means 3. Signal conditioner circuit 4 and amplifier means 3 are as described in US Pat N° 5,154,171.

The example herein refers to one of the various possible impedance methods. ESZ, a marker of end-systolic volume (residual volume) is used both as an index of right ventricular contractility and as an indicator of increased pulmonary artery pressure (a marker of left ventricular end-diastolic pressure). ESZ is measured in 5 and values are stored in a short-term moving average register 6 and a long term moving average register 7. A CPU 8 incorporating memory and software calculates the ratio between the short-term over the long-term moving averages (ratio of registers 6 and 7). If said ratio exceeds a programmable threshold value (to be assessed clinically in each patient), and lasts more than a programmable time as measured by timer circuit 9, a signal is processed according to one or more of the following options: a) reducing pacing rate to diminish myocardial oxygen demands; b) send a warning signal to the doctor or hospital via remote monitoring systems; c) produce an audible or vibratory signal to warn the patient, d) activate a drug-delivery system and e), store the time and duration of the event in register 10. Output means 11 to deliver pacing pulses to intracardiac electrodes 2. QRS sensing amplifier 12 to implement conventional pacemaker demand function. Telemetry means 13 to communicate with external programmer and/or monitoring device. Event register 9 may also have the capability to transmit by telemetry or in real time event markers detectable by surface ECG.

Now, the ischemia detection algorithm shall be described in detail:

The impedance sensor continuously measures ESZ, and the output is fed simultaneously to a long term moving average register (which stores the value of ESZ averaged over several minutes, a programmable variable) and to a short term moving average register (which stores the value of ESZ of the last 30 seconds, also a programmable feature). The short/long term average ratio (S/L) is calculated and the resulting value reaches a decision node where if the ratio is smaller than a programmable value, there is indication of a significant and abrupt increase in ESZ, consistent with depressed contractility, or with a rise of LVEDP associated with ischemia. In this case, the long term average register is stopped, holding the last value as a future reference (at the end of ischemia), and an elapsed-time ischemia timer is initiated. If the duration of ischemia exceeds a programmable value, the pacemaker takes an action, as defined previously. In this example, the ischemia warning is stored in the pacemaker memory register 10 (Fig. 2). If ischemia detection time is shorter than a preselected threshold, the long term/short-term moving averages are restarted and the device is reset.

Thus, in order for ESZ change to be attributable to ischemia, the following two conditions must be met: 1. It must present a certain rate of change (a certain amount over a certain time). This is determined by the SA/LA ratio. The ischemia signal should also persist for at least a minimum programmable time (usually 1 to 3 minutes). This is determined by ischemia timer 9.

If above two conditions are met, ischemia is suspected and a warning flag is set.

## Claims

1. An ischemia detection apparatus comprising:
- an impedance measuring stage (5) being connected to an electrode lead connector, said electrode lead connector being adapted to be connected to at least two intracardiac and/or epicardial electrodes and to produce an impedance signal indicative of a measured impedance between said at least two electrodes, and
- an ischemia detector (8) connected to the impedance measuring stage and a memory for impedance signal values, the ischemia detector being adapted to evaluate the impedance signal by determining a change in endsystolic impedance consistent with ischemia and to develop a control signal indicative of ischemia the ischemia detector comprising a comparator, said comparator being adapted to compare an actual endsystolic impedance value to a stored impedance value, **characterized in that** the comparator is connected to a timer (9), said timer being adapted to determine the duration of a time period during which the ratio or the difference between the actual endsystolic impedance value and the stored impedance value exceeds a preset threshold value.

2. Ischemia detection apparatus according to claim 1, wherein the ischemia detector is adapted to determine an endsystolic impedance value for cardiac cycle by determining the maximum impedance measured by the impedance measuring stage during a cardiac cycle.

3. Ischemia detection apparatus according to claim 1, wherein the ischemia detector comprises a long term averaging stage being adapted to determine a long term average value from a plurality of endsystolic impedance values, the long term averaging stage being connected to a long term endsystolic impedance average memory.

4. Ischemia detection apparatus according to claim 1, wherein the ischemia detector comprises a short term averaging stage being adapted to determine a short term average value from a plurality of endsystolic impedance values, the short term averaging stage being connected to a short term endsystolic impedance average memory.

5. Ischemia detection apparatus according to claim 3, wherein the stored impedance value is long term endsystolic impedance average value.

6. Ischemia detection apparatus according to claim 4, wherein the actual impedance value is the short term endsystolic impedance average value.

7. Ischemia detection apparatus according to claim 5 or 6, wherein the comparator is adapted to determine whether or not the ratio or the difference between the actual endsystolic impedance value and the stored impedance value exceeds a preset threshold value.

8. Ischemia detection apparatus according to claims 1 to 7, wherein the apparatus comprises a memory for storing the impedance sensor values and/or waveform and/or the ischemia detector output signals and transmit them via telemetry to the pacemaker programmer, to a patient warning device, a home monitoring device or the doctor's office.

9. Ischemia detection apparatus according to claims 1 to 8, wherein the apparatus comprises a pulse generator and a controller adapted for applying said control signal sensitive to ischemia to the pulse generator and/or drug delivery system to cause the pulse generator to generate pacing pulses to be applied to a pacing electrode to reduce myocardial oxygen consumption, and alleviate ischemia.

10. Ischemia detection apparatus according to claims 1 to 11, wherein the apparatus comprises a drug delivery system and a controller adapted for applying said control signal sensitive to ischemia to the drug delivery system to cause the drug delivery system to deliver a drug to reduce myocardial oxygen consumption, and alleviate ischemia.

11. Ischemia detection apparatus according to claims 1 to 10, **characterized in that** the apparatus is a VVI, DDD or multichamber cardiac pacemaker, comprising:
a) an electronic pulse generator having variable timing means for determining rate at which pacing pulses are delivered to the heart, guided by a primary rate responsive sensor means,
b) means for applying said pulses to the heart,
c) memory means for storing short term moving average values of the ischemia sensor,
d) memory means for storing long term moving average values of the ischemia sensor,
e) means responsive to said short and long term moving average values to compute a ratio,
f) timing means to measure time over which said ratio exceeds a preprogrammed time limit,
g) computing means to establish ischemia criteria based on said ratio and said time limit, and
h) control means to reduce primary sensor rate if ischemia criteria are met.

12. Ischemia detection apparatus according to claim 11, wherein the apparatus comprises telemetry means to communicate with external programmer and to send the ischemia signal to a programmer and/or to a device for displaying a surface electrocardiogram to display the ischemia signal together with the electrocardiogram.

13. Ischemia detection apparatus according to claims 1 to 12, wherein said ischemia detector is adapted to generate a ischemia signal proportional to myocardial ischemia.

14. Ischemia detection apparatus according to claim 13, wherein the apparatus comprises control means responsive to ischemia sensor signals alone or in combination with other detected parameters.

## Patentansprüche

1. Ischämiedetektionsvorrichtung, umfassend:
- einen Impedanzmesstisch, der mit einem Elektrodenleitungsverbinder verbunden ist, wobei der Elektrodenleitungsverbinder dazu geeignet ist, mit zumindest zwei intrakardialen und/oder epikardialen Elektroden verbunden zu werden und ein Impedanzsignal zu erzeugen, das für eine gemessene Impedanz zwischen den zumindest zwei Elektroden indikativ ist, und
- einen Ischämiedetektor, der mit dem Impedanzmesstisch und einem Speicher für Impedanzsignalwerte verbunden ist, wobei der Ischämiedetektor dazu geeignet ist, das Impedanzsignal durch Bestimmen einer mit Ischämie übereinstimmenden Veränderung der endsystolischen Impedanz zu bewerten und ein für Ischämie indikatives Steuersignal (Ischämiesignal) zu bilden, wobei der Ischämiedetektor einen Vergleicher umfasst, wobei der Vergleicher dazu geeignet ist, einen tatsächlichen endsystolischen Impedanzwert mit einem gespeicherten Impedanzwert zu vergleichen, **dadurch gekennzeichnet, dass** der Vergleicher mit einem Zeitmesser verbunden ist, wobei der Zeitmesser dazu geeignet ist, die Dauer eines Zeitraums zu bestimmen, während dessen das Verhältnis oder die Differenz zwischen dem tatsächlichen endsystolischen Impedanzwert und dem gespeicherten Impedanzwert den vorgegebenen Schwellenwert überschreitet.

2. Ischämiedetektionsvorrichtung nach Anspruch 1, worin der Ischämiedetektor dazu geeignet ist, einen endsystolischen Impedanzwert für den Herzzyklus durch Bestimmen der Maximalimpedanz, die vom Impedanzmesstisch während eines Herzzyklus gemessen wird, zu bestimmen.

3. Ischämiedetektionsvorrichtung nach Anspruch 1, worin der Ischämiedetektor einen Langzeit-Mittelwertbildungstisch umfasst, der dazu geeignet ist, aus einer Vielzahl an endsystolischen Impedanzwerten einen Langzeit-Mittelwert zu bestimmen, wobei der Langzeit-Mittelwertbildungstisch mit einem Speicher für endsystolische Langzeit-Impedanzmittelwerte verbunden ist.

4. Ischämiedetektionsvorrichtung nach Anspruch 1, worin der Ischämiedetektor einen Kurzzeit-Mittelwertbildungstisch umfasst, der dazu geeignet ist, aus einer Vielzahl an endsystolischen Impedanzwerten einen Kurzzeit-Mittelwert zu bestimmen, wobei der Kurzzeit-Mittelwertbildungstisch mit einem Speicher für endsystolische Kurzzeit-Impedanzmittelwerte verbunden ist.

5. Ischämiedetektionsvorrichtung nach Anspruch 3, worin der gespeicherte Impedanzwert der endsystolische Langzeit-Impedanzmittelwert ist.

6. Ischämiedetektionsvorrichtung nach Anspruch 4, worin der tatsächliche Impedanzwert der endsystolische Kurzzeit-Impedanzmittelwert ist.

7. Ischämiedetektionsvorrichtung nach Anspruch 5 oder 6, worin der Vergleicher dazu geeignet ist, zu bestimmen, ob das Verhältnis oder die Differenz zwischen dem tatsächlichen endsystolischen Impedanzwert und dem gespeicherten Impedanzwert einen vorgegebenen Schwellenwert überschreitet oder nicht.

8. Ischämiedetektionsvorrichtung nach einem der Ansprüche 1 bis 7, worin die Vorrichtung einen Speicher zum Speichern der Impedanzsensorwerte und/oder -wellenform und/oder der Ischämiedetektorausgangssignale umfasst und diese via Telemetrie an die Schrittmacher-Programmiervorrichtung, an eine Patientenwarnvorrichtung, eine Heimüberwachungsvorrichtung oder an die Arztpraxis überträgt.

9. Ischämiedetektionsvorrichtung nach einem der Ansprüche 1 bis 8, worin die Vorrichtung einen Impulsgeber und eine Steuervorrichtung umfasst, die dazu geeignet ist, das gegenüber Ischämie empfindliche Steuersignal an den Impulsgeber und/oder ein Medikamentenabgabesystem anzulegen, um den Impulsgeber zur Erzeugung von Stimulationsimpulsen zu veranlassen, die an eine Stimulationselektrode anzulegen sind, um den myokardialen Sauerstoffverbrauch zu senken und die Ischämie zu mindern.

10. Ischämiedetektionsvorrichtung nach einem der Ansprüche 1 bis 9, worin die Vorrichtung ein Medikamentenabgabesystem und eine Steuervorrichtung umfasst, die dazu geeignet ist, das gegenüber Ischämie empfindliche Steuersignal an das Medikamentenabgabesystem anzulegen, um das Medikamentenabgabesystem zur Abgabe eines Medikaments zu veranlassen, um den myokardialen Sauerstoffverbrauch zu senken und die Ischämie zu mindern.

11. Ischämiedetektionsvorrichtung nach einem der Ansprüche 1 bis 10, **dadurch gekennzeichnet, dass** die Vorrichtung ein VVI-, DDD- oder Mehrkammer-Herzschrittmacher ist, der Folgendes umfasst:
a) einen elektronischen Impulsgeber mit variablen Taktgebermitteln zur Bestimmung der Rate, mit der Stimulationsimpulse an das Herz abgegeben werden, gelenkt von einem gegenüber der Primärrate responsiven Sensormittel,
b) Mittel zum Anlegen der Impulse an das Herz,
c) Speichermittel zum Speichern der gleitenden Kurzzeit-Mittelwerte des Ischämiesensors,
d) Speichermittel zum Speichern der gleitenden Langzeit-Mittelwerte des Ischämiesensors,
e) Mittel, die gegenüber den gleitenden Kurz- und Langzeit-Mittelwerten responsiv sind, um ein Verhältnis zu berechnen,
f) Zeitmessmittel zum Messen der Zeit, während der das Verhältnis eine vorprogrammierte Zeitbegrenzung überschreitet,
g) Berechnungsmittel zum Aufstellen von Ischämiekriterien auf der Grundlage des Verhältnisses und der Zeitbegrenzung, und
h) Steuermittel zum Senken der Primärsensorrate, wenn die Ischämiekriterien erfüllt werden.

12. Ischämiedetektionsvorrichtung nach Anspruch 11, worin die Vorrichtung Telemetriemittel umfasst, um mit einer externen Programmiervorrichtung zu kommunizieren und das Ischämiesignal an eine Programmiervorrichtung und/oder an eine Vorrichtung zum Anzeigen eines Oberflächenelektrokardiogramms zu senden, um das Ischämiesignal gemeinsam mit dem Elektrokardiogramm anzuzeigen.

13. Ischämiedetektionsvorrichtung nach einem der Ansprüche 1 bis 12, worin der Ischämiedetektor dazu geeignet ist, ein zu Myokardischämie proportionales Ischämiesignal zu erzeugen.

14. Ischämiedetektionsvorrichtung nach Anspruch 13, worin die Vorrichtung Steuermittel umfasst, die gegenüber Ischämiesensorsignalen allein oder in Kombination mit anderen detektierten Parametern responsiv sind.

## Revendications

1. Un appareil de détection d'ischémie comprenant :
- Une platine de mesure d'impédance étant reliée à un connecteur de raccordement d'électrodes, ledit connecteur de raccordement d'électrodes étant indiqué pour être relié à au moins deux électrodes intracardiaques et/ou épicardiques et pour produire un signal d'impédance indiquant une impédance mesurée entre lesdites au moins deux électrodes, et
- un détecteur d'ischémie relié à la platine de mesure d'impédance et une mémoire pour les valeurs de signal d'impédance, le détecteur d'ischémie étant indiqué pour évaluer le signal d'impédance en déterminant un changement d'impédance télédiastolique correspondant à l'ischémie et pour développer un signal de contrôle indiquant l'ischémie (signal d'ischémie), le détecteur d'ischémie comprenant un comparateur, ledit comparateur étant indiqué pour comparer une valeur d'impédance télédiastolique réelle avec une valeur d'impédance mémorisée, **caractérisé en ce que** le comparateur est relié à une minuterie, ladite minuterie étant indiquée pour déterminer la durée d'une période de temps durant laquelle le rapport ou la différence entre la valeur d'impédance télédiastolique réelle et la valeur d'impédance mémorisée dépasse la valeur seuil prédéfinie.

2. Un appareil de détection d'ischémie selon la revendication 1, où le détecteur d'ischémie est indiqué pour déterminer une valeur d'impédance télédiastolique par cycle cardiaque en déterminant l'impédance maximale mesurée par la platine de mesure d'impédance durant un cycle cardiaque.

3. Un appareil de détection d'ischémie selon la revendication 1, où le détecteur d'ischémie comprend une platine déterminant la moyenne à long terme étant indiquée pour déterminer une valeur moyenne à long terme à partir d'une pluralité de valeurs d'impédance télédiastoliques, la platine déterminant la moyenne à long terme étant reliée à une mémoire moyenne d'impédance télédiastolique à long terme.

4. Un appareil de détection d'ischémie selon la revendication 1, où le détecteur d'ischémie comprend une platine déterminant la moyenne à court terme étant indiquée pour déterminer une valeur moyenne à court terme à partir d'une pluralité de valeurs d'impédance télédiastoliques, la platine déterminant la moyenne à court terme étant reliée à une mémoire moyenne d'impédance télédiastolique à court terme.

5. Un appareil de détection d'ischémie selon la revendication 3, où la valeur d'impédance mémorisée est une valeur moyenne d'impédance télédiastolique à long terme.

6. Un appareil de détection d'ischémie selon la revendication 4, où la valeur d'impédance réelle est la valeur moyenne d'impédance télédiastolique à court terme.

7. Un appareil de détection d'ischémie selon la revendication 5 ou 6, où le comparateur est indiqué pour déterminer si le rapport ou la différence entre la valeur d'impédance télédistolique réelle et la valeur d'impédance mémorisée dépasse ou non une valeur seuil prédéfinie.

8. Un appareil de détection d'ischémie selon les revendications de 1 à 7, où l'appareil comprend une mémoire dans laquelle est possible mémoriser les valeurs de capteur d'impédance et/ou formes d'onde et/ou les signaux de sortie du détecteur d'ischémie et les transmet par télémétrie au programmeur de pacemaker, à un dispositif d'avertissement du patient, à un dispositif de monitorage domestique ou au bureau du médecin.

9. Un appareil de détection d'ischémie selon les revendications de 1 à 8, où l'appareil comprend un générateur d'impulsions et un contrôleur indiqué pour appliquer ledit signal de contrôle sensible à l'ischémie à un générateur d'impulsions et/ou à un système d'administration de médicaments pour faire en sorte que le générateur d'impulsions produise des impulsions de stimulation qui doivent être appliquées à une électrode de stimulation pour réduire la consommation d'oxygène myocardique, et réduire l'ischémie.

10. Un appareil de détection d'ischémie selon les revendications de 1 à 9, où l'appareil comprend un système d'administration de médicaments et un contrôleur indiqué pour appliquer ledit signal de contrôle sensible à l'ischémie au système d'administration de médicaments pour faire en sorte que le système d'administration de médicaments administre un médicament pour réduire la consommation d'oxygène myocardique, et réduire l'ischémie.

11. Un appareil de détection d'ischémie selon les revendications de 1 à 10, **caractérisé en ce que** l'appareil est un pacemaker inihibé par QRS, un pacemaker DDD ou pacemaker à plusieurs chambres, comprenant :
a) un générateur d'impulsions électronique présentant un dispositif de temporisation variable apte à déterminer un taux auquel des impulsions de stimulation sont transmises au coeur, guidé par des moyens de capteur répondant au taux primaire,
b) des moyens pour l'application desdites impulsions au coeur,
c) des moyens de mémoire pour mémoriser des valeurs moyennes de mouvement à court terme du capteur d'ischémie,
d) des moyens de mémoire pour mémoriser des valeurs moyennes de mouvement à long terme du capteur d'ischémie,
e) des moyens répondant auxdites valeurs moyennes de mouvement à long terme et à court terme pour calculer un taux,
f) des moyens de temporisation pour mesurer le temps durant lequel ledit taux dépasse une limite de temps préprogrammé,
g) des moyens de calcul pour établir des critères d'ischémie basés sur ledit taux et ladite limite de temps, et
h) des moyens de contrôle pour réduire le taux de capteur primaire si les critères d'ischémie sont satisfaits.

12. Un appareil de détection d'ischémie selon la revendication 11, où l'appareil comprend des moyens de télémétrie aptes à communiquer avec un programmeur extérieur et à envoyer le signal d'ischémie à un programmeur et/ou à un dispositif apte à afficher un électrocardiogramme de surface pour afficher le signal d'ischémie avec l'électrocardiogramme.

13. Un appareil de détection d'ischémie selon les revendications de 1 à 12, où ledit détecteur d'ischémie est indiqué pour générer un signal d'ischémie proportionnel à l'ischémie myocardique.

14. Un appareil de détection d'ischémie selon la revendication 13, où l'appareil comprend des moyens de contrôle répondant aux signaux de capteur d'ischémie seuls ou combinés avec d'autres paramètres détectés.
